(19)

Europäisches
Patentamt

European
Patent Office

Office européen
des brevets

(11) **EP 3 501 553 A1**

(12) **EUROPEAN PATENT APPLICATION**

(43) Date of publication:
**26.06.2019 Bulletin 2019/26**

(21) Application number: **17209876.6**

(22) Date of filing: **21.12.2017**

(51) Int Cl.:
**A61K 49/18** (2006.01)     **A61K 47/69** (2017.01)
**A61K 9/00** (2006.01)      **A61K 9/06** (2006.01)
**A61K 47/36** (2006.01)     **A61K 9/16** (2006.01)
**A61K 9/70** (2006.01)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD TN**

(71) Applicants:
• **Association for the Advancement of Tissue
  Engineering and Cell based Technologies &
  Therapies (A4TEC) - Associação
  Braga (PT)**
• **Mossakowski Medical Research Centre Polish
  Academy
  of Sciences
  02-106 Warsaw (PL)**
• **Uniwersytet Warmi Sko-Mazurski W Olsztynie
  10-719 Olsztyn (PL)**
• **FMC Biopolymer AS
  1337 Sandvika (NO)**

(72) Inventors:
• **Joaquim Miguel, Antunes Correia  de Oliveira
  4715-178 Braga (PT)**
• **Sílvia Cristina, Araújo Vieira
  4705-010 Braga (PT)**
• **Maria Eduarda Moreira, Pinheiro dos Santos
  Oliveira
  4710-229 Braga (PT)**
• **Joana Catarina, da Silva Correia de Oliveira
  4715-178 Braga (PT)**

• **Rui Luís, Gonçalves dos Reis
  4250-242 Porto (PT)**
• **Piotr, Walczak
  Baltimore, 21286 (US)**
• **Izabela, Malysz-Cymborska
  10-768 Olsztyn (PL)**
• **Dominika, Golubczyk
  10-692 Olsztyn (PL)**
• **Lukasz, Kalkowski
  11-041 Warkaly (PL)**
• **Malgorzata, Majchrzak
  99-300 Kutno (PL)**
• **Miroslaw, Janowski
  Baltimore, MD Maryland 21204 (US)**
• **Paulina Natalia, Strymecka
  84-312 Lebunia (PL)**
• **Luiza, Stanaszek
  01-321 Warsaw - POLAND (PL)**
• **Barbara, Lukomska
  02-791 Warsaw (PL)**
• **Terje, Svendsen
  1385 Asker (NO)**
• **Lise Cathrine, Asdahl
  3478 Nærsnes (NO)**
• **Henriette Elisabeth Myhr, Sætrang
  1337 Sandvika (NO)**

(74) Representative: **Patentree
  Edificio Net
  Rua de Salazares, 842
  4149-002 Porto (PT)**

(54) **HYDROGEL COMPRISING MANGANESE, METHODS AND USES THEREOF**

(57)    The present disclosure relates to biocompatible ionically crosslinked hydrogel polymers comprising polysaccharides such as alginate, hyaluronic acid, gellan gum or its derivatives and manganese ions for use in medicine, in particular for imaging purposes.

Therefore, the hydrogel present disclosure is useful for spatio-temporal control of cells/drugs delivery in a wide range of therapeutic applications.

EP 3 501 553 A1

## Description

## Technical field

The present disclosure relates to a hydrogel comprising manganese, methods and use thereof. The hydrogel described in the present disclosure is suitable for use in medicine, in particular as an imaging agent.

## Background

Hydrogels are becoming very attractive biomaterial scaffolds to facilitate local cell and drug delivery. The imaging of hydrogel placement both in real-time as well as long-term enables more precise administration of them as well as to follow their fate. We describe the polymer-based structures, which undergo gelation through ionic cross-linking.

Gellan gum is a linear anionic heteropolysaccharide secreted by the bacteria Sphingomonas elodea. Its molecular structure is based in one repeating unit consisting of glucose-glucuronic acid-glucose-rhamnose. In the native form, or high acyl form, two types of acyl substituents are present: acetyl and L-glyceryl. Low acyl gellan gum is obtained through alkaline hydrolysis of native gellan gum, which removes both acyl residues. Methacrylated Gellan Gum (GG-MA) is obtained through chemical modification of the native low acyl GG. While Low- and high-acyl GG are not soluble in water at room temperature (RT), it is possible to dissolve GG-MA at RT and neutral pH, making it more user- and cell-friendly when cell encapsulation is envisioned. All the above-mentioned forms of gellan gum form hydrogels in presence of metallic ions, typically by the addition of CaCl2 and/or Phosphate Buffer Saline (PBS). Indeed, gellan gum (GG) hydrogels have been successfully used for several applications including intervertebral disc (IVD) regeneration [1], nanoparticles coating [2], bioprinting of brain-like tissues [3], among others [4]. Nevertheless, other cations can be explored and used to form GG-based hydrogels. Herein, the application of MnCl2 to physically crosslink the material is disclosed, or further applications in biomedical field, including MRI in vivo tracking using T1-weighted imaging for MRI [5].

GG can be blended with other polymers, including alginate and hyaluronic acid among others. Additionally, GG is acid and heat resistant and has a free carboxylic group per repeating unit. The presence of this carboxylic group confers to GG a negative charge at neutral pH (pKa=3.1) allowing the interaction of gellan gum with positively charged polymers to prepare polyelectrolyte complex systems.

Alginate is a polysaccharide typically extracted from the brown seaweed, or from the bacteria Azotobacter or Pseudomonas, and contains a linear copolymer block consisting of $\beta$-D-mannuronic (M) and $\alpha$-L-guluronic acid (G) monomers. The percentage of these monomers varies depending on the source of alginate and will influence the mechanical/physical properties of the produced materials owed to their involvement in the crosslinking reaction. Alginate hydrogels, for example, are produced through ionic crosslinking (e.g. $Ca^{2+}$, $Ba^{2+}$ and $Mn^{2+}$) and added cations will bind to the G or M blocks of the alginates, making the hydrogels stiffer if the alginate contains more G blocks and softer if they have higher percentage of M blocks[6]. The M and G ratio also influences the pore size and distribution, for example if the alginate contains higher G/M blocks ratio the hydrogels' pores will be bigger and vice-versa, allowing the tailoring or adjustment of the alginate to the intended purposes [7]. Moreover, alginates present an outstanding biocompatibility and biodegradability making it appealing for tissue engineering or drug delivery purposes [8]. Alginates can be produced in the form of hydrogels, macroporous fibres [8] and microbeads [9] (e.g. for cell encapsulation), nanoparticles [10] (e.g. for drug delivery) and microspheres and/or hydrogels for imaging purposes [11]. Numerous ions can be used for imaging studies, but few studies focused on Manganese ($Mn^{2+}$) for crosslinking purposes because the formed gels are unstable, however the slow release of $Mn^{2+}$ can be useful for Manganese-enhanced magnetic resonance imaging (MEMRI). Mn2+ can be used as an intracellular contrast agent in T1-weighted imaging to image cells, tissues and their in vivo activity, and its paramagnetic properties and short T1 relaxation time allows to obtain high contrast T1-weighted MRI [12].

Hyaluronic acid (HA) is a non-sulphated anionic glycosaminoglycan, found in the extracellular matrix of many body parts, composed by alternate units of the disaccharide $\beta$-1,4-D-glucuronic acid- $\beta$-1,3-N-acetyl-D-glucosamine. HA is a material of increasing significance to bioengineering and biomaterials science and is finding applications in wide ranging areas from cosmetics to immunomodulation. Its properties, both physical and chemical are extremely attractive for various technologies related to body repair.

These facts are disclosed in order to illustrate the technical problem addressed by the present disclosure.

## General Description

A diversity of imaging techniques are available to allow the medic specialist to diagnose disease or monitor therapeutic/surgical interventional procedures. Namely, ultrasound, scintigraphy, positron emission tomography (PET), single photon emission computed tomography (SPECT), X-ray, computed tomography (CT).

An aspect of the present disclosure relates to the use of biocompatible hydrogels, obtained by ionic crosslinking of different polymers including polysaccharides such as alginate, hyaluronan, gellan gum, mixtures thereof and its derivatives in the presence of manganese ions. The hydrogels of the present disclosure can be use in medicine, namely for use in photodynamic therapy or imaging techniques. Surprisingly, these Mn-based hydrogels allow spatio-temporal control of cells/drugs delivery in a wide range of therapeutic applications. Imaging moieties allow for monitoring of biomaterial administration to animals and humans in real-time during interventional procedure and to follow fate of hydrogels in vivo, using manganese enhanced-MRI (MEMRI) technique.

An aspect of the present disclosure relates to ionically crosslinked hydrogel comprising a biocompatible polysaccharide and manganese as the ionic crosslinking agent, wherein the concentration of manganese varies between 0.001-9 mM.

In an embodiment for better results, the concentration of manganese may vary between 0.01-5 mM, preferably 0.05-1 mM, more preferably 0.1 mM.

In an embodiment for better results, the ionically crosslinked hydrogel of the present disclosure may further comprise a second ionic crosslinking agent selected calcium, barium or mixtures thereof.

an embodiment for better results, the the concentration of biocompatible polysaccharide may vary between 0.25-10% (wt/$V_{hydrogel}$), preferably between 0.5-2 % (wt/$V_{hydrogel}$), preferably between 0.75-1 % (wt/$V_{hydrogel}$).

In an embodiment for better results, the biocompatible polysaccharide is may be selected from a list consisting of: alginate, hyaluronan, gellan gum, or mixtures thereof.

In an embodiment for better results, the polysaccharide is a mixture of hyaluronan and gellan gum.

In an embodiment for better results, the mass ratio of gellan gum: hyaluronan varies between 100:0 to 75:25, in particular substantially 50:50.

In an embodiment for better results, the gellan gum is a methacrylated gellan gum, preferably wherein the methacrylation degree varies from 0.01 up to 70%, preferably from 0.01 up to 50%. The measurement of the methacrylation degree may be carried out in various ways, in this disclosure the measurement of the methacrylation degree was carried out by Nuclear Magnetic Resonance (NMR) spectroscopy.

In an embodiment for better results, the hydrogel complex viscosity at 37°C varies from 0.08 to 630 Pa.s, preferably from 2 to 100 Pa.s.. The measurement of the viscosity may be carried out in various ways, in this disclosure the measurement of the viscosity was carried out by rheological analysis, using a rheometer.

Another aspect of the present disclosure relates to the use of the ionically crosslinked hydrogel of the present subject matter for use in medicine or veterinary.

In an embodiment for better results, the ionically crosslinked hydrogel of the present subject matter may be use as a drug delivery agent or as a photodynamic therapeutic agent, preferably as a magnetic resonance imaging agent, more as a magnetic resonance imaging agent.

In an embodiment for better results, the ionically crosslinked hydrogel of the present subject matter may be use in the treatment, surgery or diagnostic of a diseases by magnetic resonance imaging, in particular in the diagnosis of diseases by manganese enhanced-MRI.

In an embodiment for better results, the ionically crosslinked hydrogel of the present subject matter may be use in the treatment, surgery or diagnostic of a neoplasia or a neurodegenerative disease, in particular cancer or amyotrophic lateral sclerosis.

Another aspect of the present disclosure relates to a pharmaceutical composition comprising the ionically crosslinked hydrogel of the present subject matter and a pharmaceutical acceptable excipient and/or an active substance in a therapeutic amount.

In an embodiment for better results, the active ingredient or biomolecule is: a drug; an active ingredient, a growth hormone, a cell attractant, a drug molecule, a cell, a tissue growth promoter, a cell attractant, or combinations thereof.

In an embodiment for better results, the composition may comprise a plurality of hydrogels.

In an embodiment for better results, the composition may be administrated by oral, parenteral, intramuscular, intranasal, sublingual or intratracheal route.

In an embodiment for better results, the composition may be an injectable composition.

In an embodiment for better results, the composition may further comprise an anti-viral, an analgesic, an anti-inflammatory agent, a chemotherapy agent, a radiotherapy agent, an antibiotic, a diuretic, or mixtures thereof.

In an embodiment for better results, the composition may further comprise a filler, a binder, a disintegrant, a lubricant, or mixture thereof.

Another aspect of the present disclosure relates to a disc, fibers or microparticles comprising the ionically crosslinked hydrogel or the composition of the present subject matter.

Another aspect of the present disclosure relates to a kit comprising the ionically crosslinked hydrogel or the composition of the present subject matter.

**Brief Description of the Drawings**

The following figures provide preferred embodiments for illustrating the description and should not be seen as limiting the scope of invention.

> **Figure 1 -** Example of GG-MA based hydrogels, using different shapes. A) Hydrogels discs obtained by ionic crosslink using MnCl2 followed by immersion in buffer solution; B) GG-MA blends are injectable and can be used to form fibers; C) extrusion dripping methods can be used to prepare GG-MA based microparticles using a Mn2+-based solution to crosslink the material.

> **Figure 2 -** Reological studies on Mn-Based GG-MA hydrogels. Frequency sweep curves for hydrogels crosslinked for 5 (A) and 10 min (B). C) Shear modulus obtained for a frequency of 1 Hz. D) Phase angle average ± standard deviation for tested conditions.

**Figure 3 - Swelling and degradation behaviour of Mn-based GG-MA hydrogels.** A) Swelling of hydrogels after immersion in A PBS solution for 504 hrs (21 days); B) Weight variation between the production of the hydrogels and after 21 days of immersion in PBS. Results presented as average ± standard deviation (n=3).

**Figure 4 - Results from in vitro study using hASC-laden GGMA hydrogels.** A) Percentage of viable cells at different culturing time points; B) DNA quantification; C) Live/Dead staining, where live cells appear as green and dead cells in red; D) Cell morphology after encapsulation visualized through DAPI/Phalloidin staining, were the nuclei appears as blue and actin as red. *p<0.05, **p>0.01, *** p<0.001. n=3

**Figure 5 - Rheological studies on GG-MA-based blends crosslinked with MnCl$_2$.** Hydrogels with different rations of GG-MA/HA (100:0, 75:25 and 50:50) were prepared. Graphs represent the obtained G' and G" moduli along time. Data represented as average of at least three measurements.

**Figure 6 - Rheological studies on GG-MA-based blends crosslinked with MnCl$_2$ after 24h of incubation in aCSF.** Hydrogels with different rations of GG-MA/HA (100:0, 75:25 and 50:50) were prepared and incubated at 37ºC in aCSF for 24 hrs. Graphs represent the obtained G' and G" moduli along time for each condition tested. Data represented as average of at least three measurements.

**Figure 7 - Scanning electron microscope pictures of freeze dried hydrogels solutions.** Surface morphology of the different hydrogel formulations was assessed by scanning electron microscopy (SEM), without the addition of any ionic solution besides MnCl$_2$.

**Figure 8 - Degradation assay with and without hyaluronidase.** After being prepared, hydrogels were incubated at 37ºC, with shaking, in a solution of aCSF or aCSF with hyaluronidase (pH 7.4). Results are shown as average ± SD of three hydrogel replicas.

**Figure 9 - Maximum force needed to inject hydrogel solution into aCSF.** Maximum force (N) registered while injecting different material formulations into aCSF. Results presented as average ± SD of three different replicas. (*) p<0.05, (****) p<0.0001.

**Figure 10** - **Permeability of different hydrogels to three FITC-labelled dextran molecules (4, 20 and 70 kDa).** Different hydrogel formulations were mixed with Dextran of different molecular weights to assess their permeability. Results are presented as average ± SD of three different replicas.

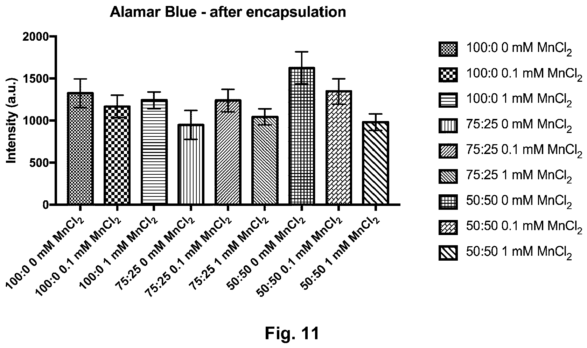

**Figure 11 - Metabolic activity of hASC after encapsulation with different GG-MA based blends crosslinked with Mn$^{2+}$.** Metabolic activity was assessed using Alamar Blue® assay. Each gel was prepared in triplicate and measurements are represented as average ± SD.

**Figure 12** - Fiber formation by extrusion into aCSF.

**Figure 13** - **hASC encapsulated in a GG-MA based fiber after 7 days of culture.** Fibers were formed using a 31 G needle coupled with a Hamilton syringe of 10 μl. Cells are stained with DAPI (blue), which stains the nuclei and the hydrogel; and Phalloidin (red), that stains cytoplasmatic actin.

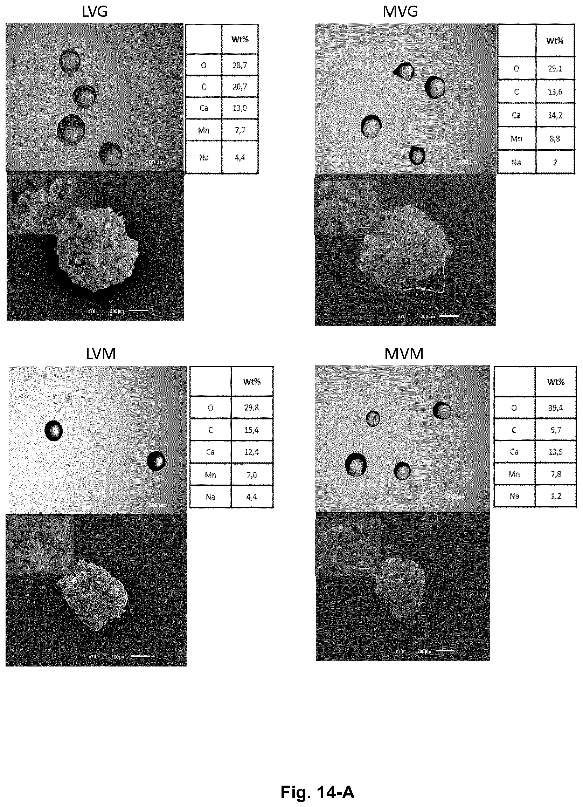

**Figure 14 A-B-** SEM and EDS analysis of the Mn-microparticles produced with the different alginates.

**Figure 15 -** T1 weighted MRI of Mn$^{2+}$ phantom preparations using clinical 3T scanner.

**Figure 16 -** MRI analysis of the stability of MnCl$_2$ in alginate hydrogels in vitro.

**Figure 17 -** T1-weightened MRI scans of intrathecal Mn$^{2+}$ based alginate delivery.

## Detailed Description

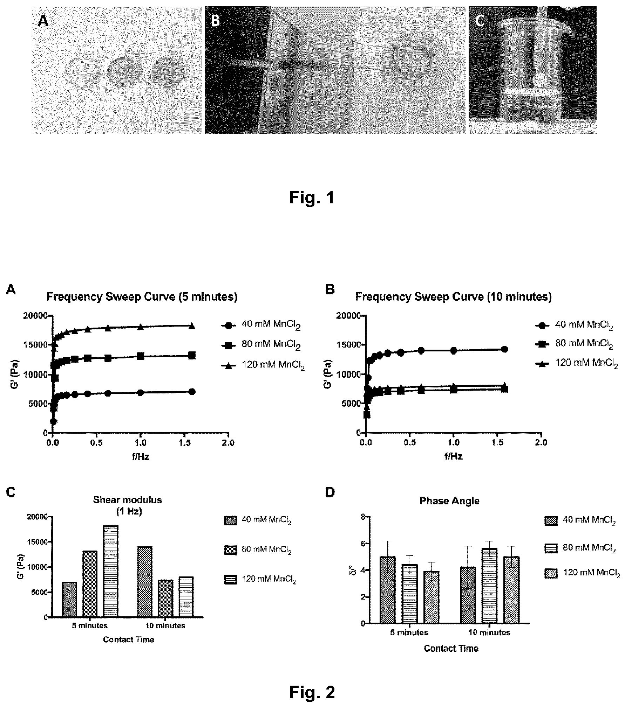

Hydrogels using different polysaccharides, including gellan gum and its derivatives, alginate, hyaluronic acid or mixtures thereof, can be prepared using different morphologies including discs, fibers or microparticles (Figure 1).

In an embodiment, discs can be prepared using hydrogels solution can be prepared by mixing 1% GG-MA with MnCl$_2$ solutions to obtain hydrogels with different concentration of Mn$^{2+}$ (0, 0.1, 1 mM of Mn$^{2+}$). These solutions were then mixed with 1% sodium hyaluronate in ratio 75:25 and 50:50 and poured into silicon molds. The disc shape is obtained after contact with ionic solutions/buffers such as artificial cerebrospinal fluid (aCSF), Phosphate buffer saline (PBS), simulated body fluid (SBF) among others. For higher concentrations of Mn$^{2+}$ (higher than 20 mM) discs can be formed without addition of ionic solutions.

In an embodiment, fibers can be prepared using the abovementioned mixture extruded directly into aCSF or another buffer. The ions present in this solution can further crosslink the GG present in gel solutions, giving a fiber shape to the injected gel.

In an embodiment, microparticles can be prepared using alginate, gellan gum and gellan gum with hyaluronic acid at desired concentration and then extruded into a solution of $CaCl_2$ and $MnCl_2$.

In an embodiment, manganese-based GG-MA hydrogels of the present disclosure can be prepared using methacrylated gellan gum (GG-MA) produced in house was crosslinked with different divalent ions to obtain a hydrogel. Briefly, GG-MA powder was dissolved in distilled water under constant mixing, upon complete dissolution, with a final concentration of 1% w/v. Then, 100 $\mu$L of GG-MA solution were dispensed into cylindrical PDMS molds and crosslinked with 50$\mu$L of $MnCl_2$ (40, 80 and 120 mM), for 10 min at room temperature. The obtained hydrogels were then detached from the PDMS mold and washed with PBS.

In an embodiment, swelling and degradation studies were carried out for GG-MA hydrogels. For that, hydrogels were frozen at -80$\underline{o}$C and freeze-dried. Then, the obtained structures were weighted (initial dry weight, $W_{id}$), hydrated and incubated with PBS (pH 7.4) at 37$\underline{o}$C with a continuous shaking of 60 rpm, up to 21 days, in triplicate. At defined time-points (1 hrs, 3 hrs, 6 hrs, 8 hrs, 1 day, 3 days, 1 week, 2weeks and 3 weeks) scaffolds were removed from the incubation solution and weighted (Wet weight, $W_w$), after removal of PBS excess. Then, water uptake (Wu) was calculated using Equation 1:

$$W_U(\%) = \frac{W_w - W_{di}}{W_{di}} \times 100$$

After three weeks of incubation, scaffolds were frozen again at -80$\underline{o}$C and freeze-dried. The resulting structures were then weighted, to obtain the final dry weight ($W_{fd}$). Equation 2 was used to estimate the degradation of the hydrogels:

$$W_l(\%) = \frac{W_{fd} - W_{id}}{W_{id}} \times 100$$

In an embodiment, rheological analyses were performed using a Kinexus Pro+ rheometer (Malvern Instruments, UK), using the acquisition software rSpace. The measuring system was equipped with stainless steel (316 grade) parallel plates: the upper measurement geometry plate, with 8 mm of diameter, and the 20 mm lower pedestal with roughened finish (to prevent sample slippage and resulting errors on the experiments). The effect of the contact time between the polymeric solution and the crosslinker was also analysed. For that, hydrogels were crosslinked for 5 or 10 min and then frequency sweep curves were obtained from $0.01^{-1}$ to 100 s$^{-1}$, being acquired 5 samples per decade, at 37°C. All plots are the average of at least 3 experiments.

In an embodiment, human adipose-derived stem cells (hASCs) were isolated from Infrapatellar Fat Pad (human Hoffa's Body (HHB)) obtained from a male, and approved by the Ethics Committee of University of Minho. All samples were processed within 24 hrs after the surgical procedure. The undifferentiated cells were cultured and expanded under basal condition, using Minimum Essential alpha Medium (alpha-MEM, Sigma, USA), supplemented with 10% (v/v) fetal bovine serum (ThermoFisher Scientific, reference 10270106, EU approved) and 1% Antibiotic-Antimycotic liquid prepared with 10,000 U.mL$^{-1}$ penicillin G sodium, 10,000 mg mL$^{-1}$ streptomycin sulfate, and 25 mg.mL$^{-1}$ amphotericin B as FungizoneVR in 0.85% saline (Life Technologies, Carlsbad, CA), until passages 1. The cells were used in passages 2.

In an embodiment, manganese-based hASC-laden GGMA hydrogels of the present disclosure can be prepared using sub-confluent cultures (90% confluence) were detached from the culture flask by using TrypLE Express (1x) with phenol red (Life Technologies, Carlsbad, CA), followed by centrifugation at 1,200 rpm for 5 min (5810R, Eppendorf, Hamburg, Germany). Then, the supernatants were discarded and the pellet mixed with 1% (w/v) GGMA solution to a final cellular density of 1x10$^6$ cells/mL. hASC-laden hydrogels were prepared by dispensing 100 $\mu$L of cell-laden GG-MA solutions into cylindrical PDMS moulds followed by the addition of 50$\mu$L of 40 mM $MnCl_2$ or 50 $\mu$L of 100 mM $CaCl_2$ (control). The moulds were then placed at 37$\underline{o}$C in a humidified atmosphere of 5% $CO_2$ in air, for 10 min. The obtained hydrogels were then detached from the PDMS mould, gently washed with PBS and transferred to 48-well plates (Corning, USA) with 500 $\mu$L of cell culture media in each well. All conditions were performed in triplicate (n=3).

In an embodiment, cell proliferation was quantified using the Quant-iT Pico-Green dsDNA Assay Kit (Life Technologies, Carlsbad, CA, USA), according to the manufacturer's instructions (n= 9). Briefly, the GGMA scaffolds were washed firstly with PBS and then mechanically destroyed for cell recovery, using 1 mL ultra-pure sterile water to lyse the cells. A thermal shock was performed at 37$\underline{o}$C for 1 hour, followed by freezing at -80 $\underline{o}$C and kept until samples analysis. For the DNA quantification readings, 28.7 $\mu$L supernatant from each vial was mixed with 71.3 $\mu$L PicoGreen working solution and 100 $\mu$L Tris-EDTA buffer. The fluorescence intensity of the samples was recorded in a microplate reader (Synergy HT; Bio-Tek, VT), with the excitation wavelength at 485/20 nm and the emission wavelength at 528/20 nm. Standard curve was prepared by using standard dsDNA solutions with different concentrations, to quantify of the DNA content in the samples. Three independent experiments were performed. Scaffolds without cell seeding were used as a negative control for fluorescent intensity correction.

In an embodiment, live-dead and DAPI-phalloidin fluorescence assays were performed at each time culture period. Calcein-AM and propidium iodide dyes were used to perform a live-dead assay. Calcein-AM is hydrolyzed by endogenous esterase into the negatively charged green fluorescent cell marker calcein, retained in the cytoplasm. PI is a membrane impermeant and only binds to DNA of dead cells. Briefly, at each time point, culture medium was removed and 500 $\mu$L of PBS containing 1 $\mu$L of calcein-AM and 0.5 $\mu$L of PI was added to each well. Samples were then incubated at 37°C for 10 min protected from light. After incubation, samples were washed three times with PBS and immediately visualized in the dark by fluorescence microscopy (Axioimage RZ1M, Zeiss, Germany). With calcein-AM, through a fluorescent filter (fluorescence excitation of 494 and emission of 517 nm), living cells appeared bright green. With PI, through a rhodamine filter (fluorescence excitation of 535 nm and emission of 617 nm), dead cells appeared bright red. Live/Dead ratio was obtained using CellTracker software (Broad Institute, Cambridge MA, USA). DAPI and phalloidin dyes were used to perform a DAPI-phalloidin assay. DAPI stains preferentially double-stranded DNA by delineating cells nuclei in blue. Phalloidin is a bicyclic peptide with selectivity to label F-actin, revealing the distribution of actin filaments in fixed cells. Prior to staining, at each time point, culture medium was removed and 10% formalin was added to each well. After 1 h at RT, formalin was removed and replaced by PBS and placed at 4$\underline{o}$C until using. For staining, 0.1% Triton X was first added for 5 min to permeabilize cells. Upon PBS washing, 1 mL of PBS containing 1 $\mu$L of DAPI and 5 $\mu$L of phalloidin was added to each well. After 45 min at RT protected from light, samples were washed three times with PBS and immediately visualized in the dark by confocal microscopy (TCS SP8, Leica). Cell nuclei appeared bright blue and F-actin filaments appeared bright red due to DAPI and phalloidin dyes, respectively.

Data was analysed by two-way ANOVA (GraphPad Prism v7 software) with the Tukey's comparisons test, and the statistical differences between samples were considered for $p < 0.05$. Cell viability data and DNA concentration were averaged and mean $\pm$ standard deviation was plotted. Statistical significance was determined by a two-tailed t-test. The critical level of statistical significance chosen was p<0.05.

Hydrogels of the present disclosure were successfully obtained for all the conditions/formulations tested. To better understand the time desired to obtain a hydrogel a rheological analysis was performed. From LVR determination, at 1Hz of frequency, a shear strain of 0.01% was defined to be correctly used on the continuing oscillatory experiments of manganese-based GG-MA gels. Frequency sweep curves obtained from oscillatory shear measurements are shown in Error! Reference source not found.-A and B, showing the dependence of storage modulus (G') upon the frequency, for gels of GG-MA in contact with the solutions by 5 min (A) and 10 min (B). Loss modulus (G") is not shown in the charts, but it was always smaller than storage modulus. G' measures the deformation energy stored during shear stress, i.e. the material stiffness.

In an embodiment, to simplify the comparison between 5 and 10 min of crosslinking, the values of G' obtained at 1 Hz were plotted together (Error! **Reference source not found.**-C). After 5 min of crosslinking, the hydrogel crosslinked with 120 mM of $MnCl_2$ showed higher gel character, while the one crosslinked with 40 mM $MnCl_2$ showed the lowest one. Surprisingly, for higher crosslinking times, i.e. 10 minutes, the hydrogel obtained with 40 mM $MnCl_2$ was the one with higher gel character, with the other two conditions resulting in weaker hydrogels, like the one crosslinked for 5 min with the lowest concentration of $MnCl_2$.

In an embodiment, the interaction strength of internal structure in a viscoelastic emulsion is measured by the magnitude of the ratio G"/G', known as the damping factor (or as loss factor). This factor, tan $\delta$, is determined by G"/G'. The damping factor is higher than 1 for viscous liquids and lower than 1 for elastic solids. This could also be measured by the correspondent phase angle ($\delta$), which is near 90$\underline{o}$ for viscous liquids (higher loss modulus - G") and near 0$\underline{o}$ for elastic solids (higher storage modulus - G'). Thus, to better understand the differences between the formulations and the used contact time, the average phase angle of each formulation (at different contact times - 5 min and 10 min) was determined. As aforementioned, the G" was always smaller than G', resulting in small values of phase angle, showing the gel character of all samples (**Error! Reference source not found.** -D). Although the hydrogel in contact for 10 min with 40 mM $MnCl_2$ appears to have the lower phase angle, thence the higher storage modulus, no significant difference was found between different conditions.

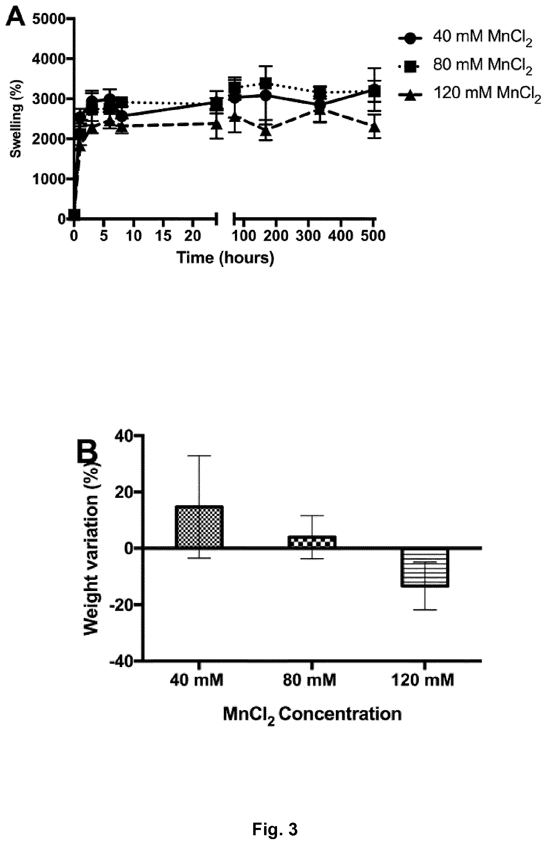

In an embodiment, from swelling studies, it was concluded that even when different concentrations of manganese were used, the swelling capacity was similar, as no significant difference was found between the different hydrogels (**Figure 3-A**). Nevertheless, hydrogels obtained with 120 mM of manganese showed a lower swelling capacity, possibly due to a higher number of crosslinking points, resulting in a closer polymeric network.

In an embodiment, regarding the weight variation caused by the immersion in PBS, the results were also statistically not significant (**Figure 3-B**). Additionally, the variations were relatively small, meaning that scaffolds remained stable after the 21 days of immersion. Interestingly, the weight increased after this incubation period for samples crosslinked with the lowest concentration of manganese. This can be due to the remaining salt inside the hydrogel network after the freeze-drying process. As the sample crosslinked with 120 mM $MnCl_2$ displayed a lower swelling capacity, meaning less inflow of salt into the hydrogel, the final weight was not affected.

In an embodiment, the first *in vitro* studies were performed using GG-MA hydrogels crosslinked with 40mM $MnCl_2$. For that, hASC-laden GG-MA were crosslinked with a solution of 40 mM $MnCl_2$ for 10 min at 37ºC (Mn-GG-MA). Cell-laden GG-MA hydrogels crosslinked with 100 mM $CaCl_2$, were used as control since it is considered a gold standard for GG-MA gelation (Ca-GG-MA). Encapsulated cells were then maintained in culture up to 7 days at 37ºC in a humidified atmosphere of 5% $CO_2$ in air.

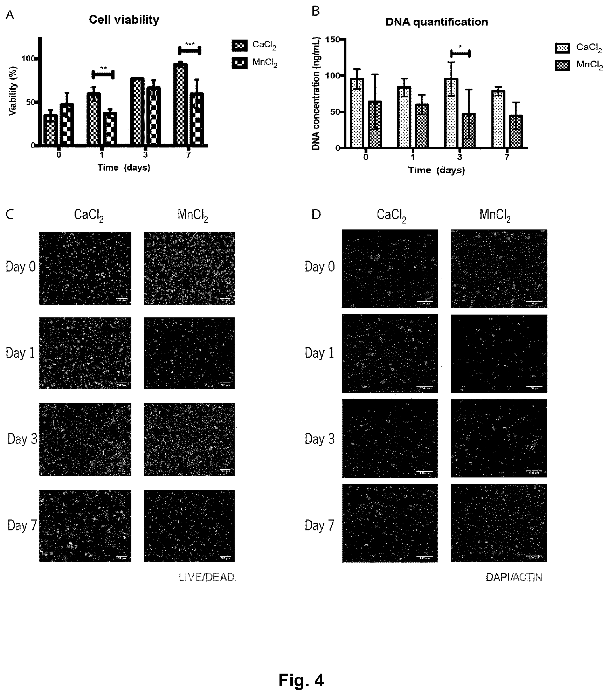

In an embodiment, using a Live/Dead staining it was possible to assess cell viability after the encapsulation process (t=0 days) and through the culture period at days 1, 3 and 7. As shown in **Figure 4**-A and C, cell viability was significantly lower when hydrogels were crosslinked with $MnCl_2$. After 7 days of culture only 59% of cells encapsulated in Mn-GG-MA hydrogels were viable, as compared to 93% of viable cells encapsulated in Ca-GG-MA hydrogels.

In an embodiment, cell proliferation was studied by accessing the amount of DNA present in individually hydrogels at each time-point. As represented in **Figure 4-B**, DNA amount did not change significantly along the 7 days of experiment showing that cells are in a non-proliferative state inside both Ca- and Mn-based hydrogels.

Once inside the gels, hASCs, acquired a round morphology, as shown by confocal images from samples stained with DAPI (nuclei, blue) and Phalloidin (actin, red). As depicted in **Figure 4-D**, the round morphology was preserved through the 7 days of experiment but cytoplasm degradation may occur, as some debris were noticed from 3 days afterwards.

Manganese-based hydrogels of the present disclosure were successfully obtained in all the conditions tested. These hydrogels have a high swelling capacity and non-degradable nature, as shown by the swelling and degradation studies. Cell-laden hydrogels were successfully obtained after GG-MA crosslinking with manganese.

In an embodiment, GG-MA/HA blends crosslinked with MnCl2 can be prepare_using manganese-based GG-MA hydrogels of the present disclosure can be prepared Methacrylated gellan gum (GG-MA) produced in house was crosslinked with different divalent ions to obtain a hydrogel. Briefly, GG-MA powder was dissolved in distilled water under constant mixing, upon complete dissolution, with a final concentration of 1% w/v. Then, $MnCl_2$ solutions were added to obtain 0.75% (w/v) GG-MA hydrogels with different concentration of $Mn^{2+}$ (0, 0.1, 1 mM of $Mn^{2+}$). These solutions were then mixed with 1% sodium hyaluronate in ratio 75:25 and 50:50 and poured into silicon molds. The final shape was obtained after contact with artificial cerebrospinal fluid (aCSF).

In an embodiment, rheological analyses of an hydrogel of GG-MA/HA blends crosslinked with MnCl2 were performed using a Kinexus Pro+ rheometer (Malvern Instruments, UK), using the acquisition software rSpace. The measuring system was equipped with stainless steel (316 grade) parallel plates: the upper measurement geometry plate, with 8 mm of diameter, and the 20 mm lower pedestal with roughened finish (to prevent sample slippage and resulting errors on the experiments). All data was collected at 37ºC and plots are the average of at least 3 experiments.

In an embodiment, surface morphology of the different hydrogels was characterized by scanning electron microscopy (SEM). For that, gels were frozen at -80ºC and then freeze-dried (LyoAlfa 10/15, Telstar). Prior to SEM visualization, samples were fixed with mutual conductive adhesive tape and coated with a thin layer of gold using a sputter coater (EM ACE600, Leica, Germany). At last, capsules were visualized using a FEI Nova NanoSEM 200 operating at 15 kV accelerating voltage.

Stability of hydrogels was studied by incubating hydrogels in aCSF solution and aCSF with hyluronidase solution with a final concentration of 2,6 U (plasma concentration). After being prepared, hydrogels were weighted (initial weight, $W_i$) and incubated in aCSF (pH 7.4) with and without enzyme, at 37ºC with shaking for 7days. At each time point (1h, 3h, 1, 3 and 7 days) samples were retrieved from aCSF solutions, weighted (wet weight, $W_w$) and placed again in solution. Then, hydrogel degradation ($W_D$) was calculated using Equation 1:

$$W_D(\%) = \frac{W_w - W_i}{W_i} \times 100$$

The possibility to extrude of GG-MA blended solutions from a 31 G needle was investigated by means of using an injectability equipment (Paralab). The measurements were performed using a 10 μL Hamilton syringe coupled with a 31 G needle. The syringe was filled with the GG-MA blends as well as distilled water. Then, each solution was injected through the needle by applying a force on the syringe piston, using a constant rate. The load applied to the piston for injecting the material through the 31 G needle was measured and recorded using an appropriate software.

In an embodiment, the permeability of different GG-MA blend discs was assessed using fluorescence-labelled molecules with different molecular weight. Fluorescein isothiocyanate-dextran (Dextran-FITC, Sigma) with different molecular weights (4, 20 and 70 kDa), were mixed with GG-MA solutions and gels were prepared as stated before. Then, each gel was incubated 7 days (168 hours) in aCSF, using a shaking waterbath at 37ºC. At different timepoints, a small amount of the supernatant was retrieved and the same amount of fresh aCSF was added to each sample. At 168 hours, gels were mechanically destroyed in 1 mL of aCSF and centrifuged. The resulting supernatant was used to calculate the concentration of FITC-labelled molecules that have remained inside the capsules. The fluorescence emission of the retrieved supernatant was measured at an excitation wavelength of 485/20 nm and at an emission wavelength of 528/20 nm, in a microplate reader (Gen 5 2.01, Synergy HT, BioTek). The final concentration of released fluorescent-labelled molecules was obtained using a standard curve with defined concentrations.

Human adipose-derived stem cells (hASCs) were isolated from Infrapatellar Fat Pad (human Hoffa's Body (HHB)) obtained from a male, and approved by the Ethics Committee of University of Minho. All samples were processed within 24 h after the surgical procedure. The undifferentiated cells were cultured and expanded under basal condition, using Minimum Essential alpha Medium (alpha-MEM, Sigma, USA), supplemented with 10% (v/v) fetal bovine serum (ThermoFisher Scientific, reference 10270106, EU approved) and 1% Antibiotic-Antimycotic liquid prepared with 10,000 U.mL-1 penicillin G sodium, 10,000 mg mL-1 streptomycin sulfate, and 25 mg.mL-1 amphotericin B as FungizoneVR in 0.85% saline (Life Technologies, Carlsbad, CA), until passages 1. The cells were used in passage 2.

In an embodiment, sub-confluent cultures (90% confluence) were detached from the culture flask by using TrypLE Express (1x) with phenol red (Life Technologies, Carlsbad, CA), followed by centrifugation at 1200 rpm for 5 min (5810R, Eppendorf, Hamburg, Germany). Then, the supernatants were discarded and the pellet mixed with the different blends of Mn-crosslinked GG-MA and HA, to a final cellular density of $1 \times 10^6$ cells/mL. hASC-laden hydrogels were prepared by dispensing 100 $\mu$L of cell-laden gel solutions into 24-well plates followed by the addition of 50$\mu$L of cell culture media. After gelation, each well was filled with 1 mL of cell culture media and plates were at 37$\underline{o}$C in a humidified atmosphere of 5% $CO_2$ in air. All conditions were performed in triplicate (n=3).

In an embodiment, cell viability was assessed using AlamarBlue® assay (Bio-Rad Laboratories), following manufacturer instructions with slight modifications. Briefly, after 24 h of incubation cell culture media was changed and new media supplemented with 20% (v/v) AlamarBlue was added. Plates were then incubated in the dark at 37$\underline{o}$C, in a humidified atmosphere of 5% $CO_2$ in air for 4 hrs. Afterwards, fluorescence was measured at an excitation wavelength of 528/20 nm and at an emission wavelength of 590/20 nm, in a microplate reader (Gen 5 2.01, Synergy HT, BioTek).

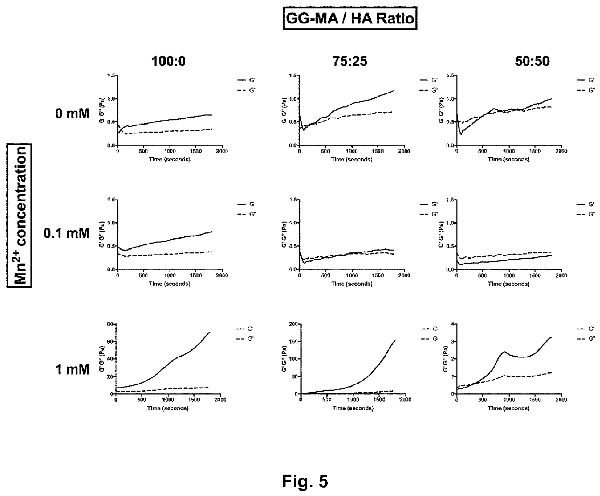

In an embodiment, after preparation, hydrogels were characterized regarding their rheological properties along time, using a single frequency of 0.1 Hz and a shear strain of 0.01 % was defined to be correctly used on the continuing oscillatory experiments. As depicted in **Figure 5,** hydrogels prepared without or with the lowest concentration of $Mn^{2+}$ (0.1 mM) have lower G' (material stiffness) are considerably weaker as compared to the ones prepared using 1 mM of $Mn^{2+}$. This confirms the interaction between $Mn^{2+}$ ions and GG-MA network, as $Mn^{2+}$ divalent cation, it interacts with GG-MA molecules, triggering its crosslinking process into a helix-coil structure. Surprisingly, when a 75:25 ratio of GG-MA/HA was used, the resulting hydrogels were stronger. The addition of a small fraction of HA allows penetration within the GG-MA network, that can still be crosslinked with $Mn^{2+}$ ions. But when the ratio increases to 50% in mass of HA, the network formed is weaker due to the presence of a higher amount of HA, that is not crosslinked by the present cations. Another interesting observation relies on the fact that G' increases with time for all the conditions tested. This can be due to a diffusion of the ion through the hydrogel matrix that consolidates its crosslink.

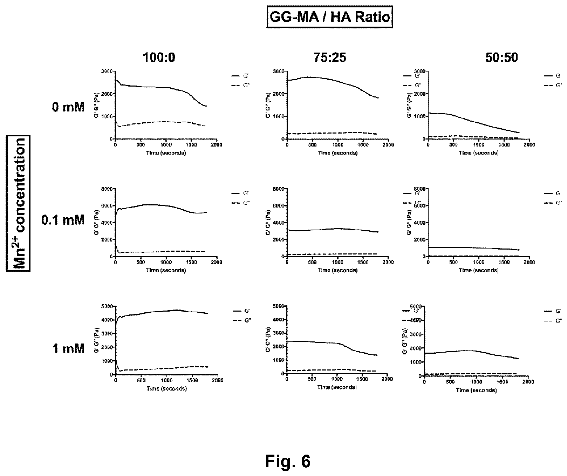

In an embodiment, the rheological properties after incubation in aCSF were also studied (**Figure 6**). For that, all hydrogels were incubated at 37$\underline{o}$C for 24h in aCSF. As expected, the G' moduli increased upon immersion in this solution, due to the presence of different cations that further crosslink the GG-MA matrix. It was also possible to conclude that the addition of HA to the network decreased the stiffness of the resulting hydrogels, as this polymer is not crosslinked ionically as GG-MA.

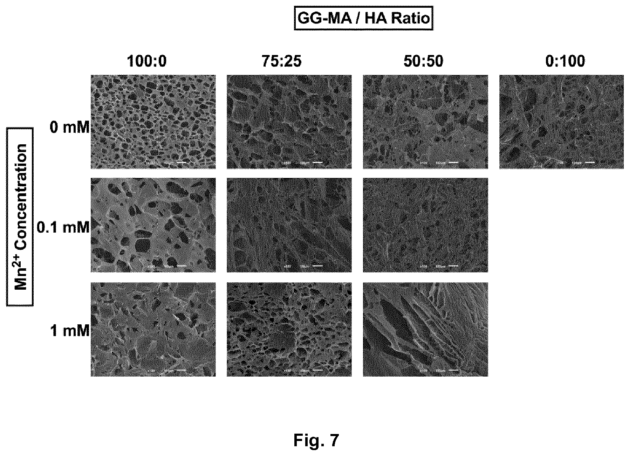

In an embodiment, morphology of gels was assessed using scanning electron microscopy (SEM). As showed in **Figure 7,** surface morphology is highly dependent of formulation. While GG-MA only hydrogels have a honeycomb-like structure, when HA is added to the mixture hydrogels show a network-like structure, that results from HA, mixed with more porous regions, resulting from GG-MA. However, for high concentrations of $Mn^{2+}$, the porous structure is predominant which can be a result of poor entrapment of HA as some of the GG-MA network is already formed and do not allow HA penetration.

In an embodiment, hydrogel stability was assessed by measuring the weight variation of hydrogels along 7 days of incubation in aCSF solution and aCSF with hyluronidase (2.6 U, plasma concentration).

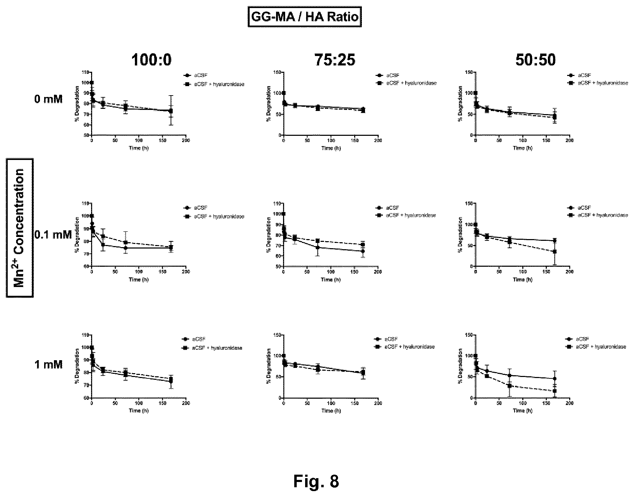

In an embodiment, gels were prepared in aCSF in silicon molds and then kept in aCSF or aCSF/hyaluronidase solution in water bath with shaking at 37°C for 7 days, to assess the degradation rate. At each time point (1, 3, 24, 72 and 168 hours) samples were retrieved from aCSF solutions, weighted and placed again in solution. As depicted in **Figure 8,** after 7 days of incubation it was possible to observe that hydrogels without HA had an 80% decrease on their initial mass, independently of $Mn^{2+}$ content and presence of hyaluronidase. For the formulation 75:25, it was observed a decrease of approximately 60% of the initial mass in all conditions. For 50:50 formulation, it was observed a 40% decrease in mass for 0 and 0.1 mM of $Mn^{2+}$ and 20% for 1 mM of $Mn^{2+}$. In all conditions tested the presence of hyaluronidase did not significantly affect the degradation rate of the gels. Therefore, one can assume that the higher degradation profile of gels prepared with HA (75:25 and 50:50) are not a consequence of enzymatic degradation of HA but a release of this polymer to solution since HA is not crosslinked but entrapped within GG-MA chains. Consequently, hydrogels with 50:50 content in GG-MA and HA had a quicker degradation. This process explains why mass of hydrogels 50:50 condition reached lowest mass after 7 days of incubation in aCSF solution. Not significant influence of hyaluronidase for 75:25 formulation could be observed because sodium hyaluronate were washed from hydrogel faster than enzyme could degrade HA.

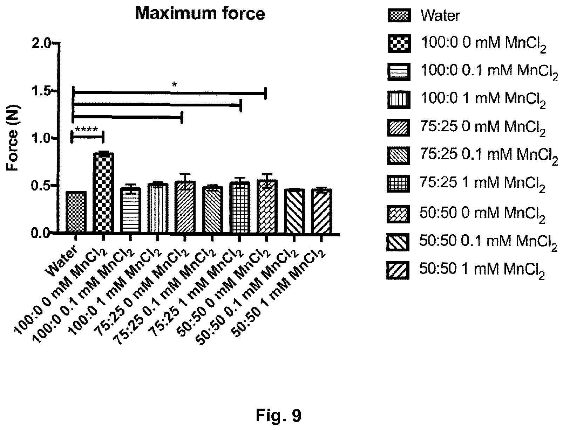

In an embodiment, injectability of hydrogels was investigated by material extrusion with Hamilton syringe coupled with a 31 G needle, into aCSF. All formulations of hydrogels are injectable, with force needed to inject gel solution being similar to the force needed to inject water, although four conditions had a significant different on maximum force used to inject the material into solution (**Figure 9**). No needle clotting was observed during this experiment. Moreover, maximal force used to inject the hydrogel in each experiment was similar for all formulations, with no significant difference observed. Surprisingly, GG-MA 0 mM $Mn^{2+}$ reached 0.8 N, which was higher value compared to the values obtained for other conditions.

In an embodiment, permeability was studied using FITC-labelled dextran molecules with three sizes: 4 kDa, 20 kDa and 70 kDa mixed with gels solutions. Gels were prepared with the addition of aCSF to form defined discs and then incubated at 37°C, with shaking, up to 7 days. At each time point, a sample of supernatant was retrieved and the same volume of fresh aCSF was added.

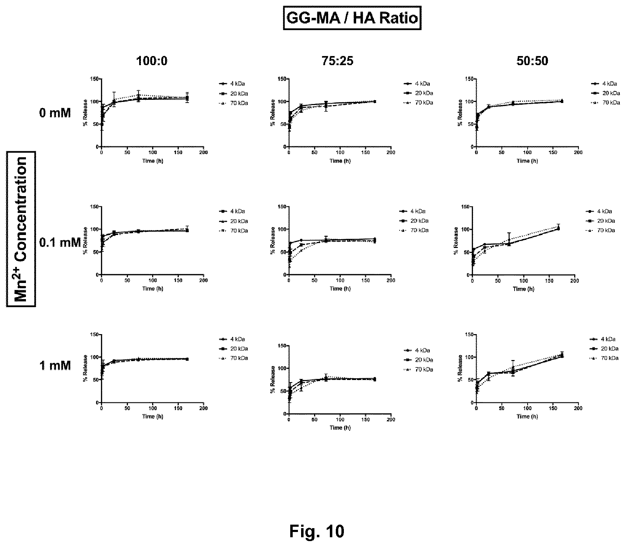

In an embodiment, as shown in **Figure 10**, $Mn^{2+}$ concentration does not affect the release profile, as hydrogels with the same GG-MA/HA ratio but different $Mn^{2+}$ concentration showed similar release trends. The only exception is the condition 75:25, where $Mn^{2+}$ appears to decrease the release of dextran from the hydrogels. However, a stronger trend is noticed when the ratio between polymers is changed. Surprisingly, hydrogels with a 75:25 ratio crosslinked with $Mn^{2+}$ did not release all their cargo, reaching a plateau at near 70%. For 100:0 and 50:50 ratios all Dextran was released but with different profiles. While hydrogels prepared with only GG-MA had a fast release for all $Mn^{2+}$ concentrations, the 50:50 blend showed a slower release profile when $Mn^{2+}$ ions were present. All hydrogels are permeable for all the molecules tested, *i.e.* small molecules with 4 kDa size and big molecules up to 70 kDa, which is of outmost importance where cells are intended to be used as delivery agents of growth factors.

In an embodiment, biocompatibility studies were performed for all hydrogels formulations, human adipose stem cells (hASC). For that, cells were finely mixed with gels solutions making up a concentration of $1x10^6$ cell/mL. Then, 100 $\mu$L of cell-laden gel solutions were dispensed in 24-wells plate followed by the addition of 50 $\mu$L of cell culture media for further crosslink. Cell viability was measured by quantifying the metabolic activity using the Alamar Blue® assay.

In an embodiment, blends of manganese-based hydrogels were successfully obtained for all the conditions and ratios tested. The obtained hydrogel solutions are easily injectable into aCSF and can be used as delivery agents of therapeutic molecules, as they have a fast release profile for small and large molecules. Cell viability assays showed that gels prepared using a concentration of 0.1 mM of $MnCl_2$ appear to be the less deleterious for cells upon cell encapsulation.

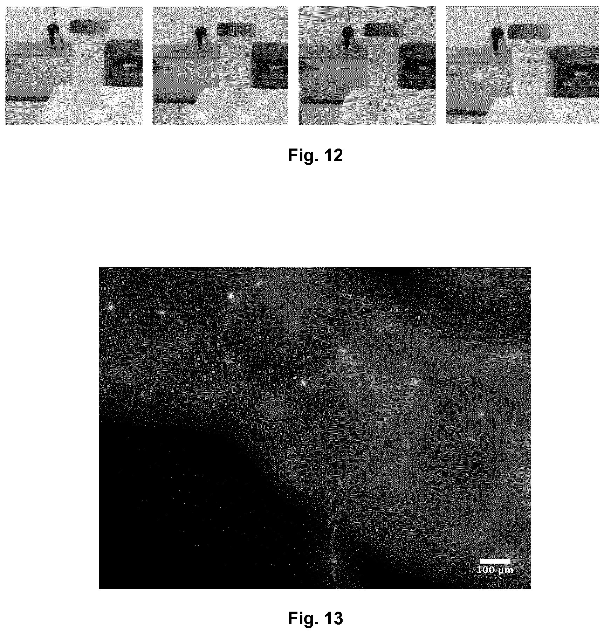

In an embodiment, GG-MA based hydrogels with different contents of $Mn^{2+}$ can be prepared as fibers by simple extrusion of the material into an ionic solution. As example, GG-MA based hydrogels, containing HA and $MnCl_2$ in different ratios were prepared and extruded from a 1 mL plastic syringe coupled with a 21G needle into aCSF, an ionic solution that mimics *in vitro* the cerebrospinal fluid present in human body. As depicted in **Figure 12,** gels were successfully extruded without needle clothing and fibers are formed, being stable after their extrusion. As fibers can be formed using different needles their diameter will be highly dependent on needle inner diameter.

In another embodiment, fibers formed with a 31 G needle (inner diameter of 133 $\pm$ 19.0 $\mu$m) origins fibers with an average diameter of 633.58 $\pm$ 33.80 $\mu$m. These fibers were used to encapsulate human adipose-derived stem cells (hASCs) up to 7 days. As depicted in **Figure 13,** cells were found inside fibers after 7 days of culture. Contrarily to cells present outside the fiber, which have spindle-like shape, encapsulated cell showed a round morphology, typical of encapsulated cells.

In an embodiment, microparticles can be formed by gravitational dripping into Mn-containing bath solutions. As example, alginates with different G/M ratios (LVG, MVG, VLVG, LVM, MVM and VLVM) can be used to produce particles. For that, they were dissolved into NaCl (0.9%), to obtained alginates at the final concentration of 1% (w/v). Then, calcium Alginate (CaM>75), at 1% (w/v) in NaCl 0.9%, mixed with 20 nM of manganese ($MnCl_2$) was used as precipitation solution. At last, 1 mL of each solution of different alginates was passed into a syringe with a 27G needle and extruded drop-wise into the precipitation solution, to obtain the different $MnCl_2$ microparticles. After production, each microparticle batch was passed into an Eppendorf and frozen at -80ºC for freeze-drying preparation, for further analysis with scanning electron microscope (SEM) and Energy Dispersive Spectroscopy (EDS). As shown in **Figure 1** microparticles were successfully produced for all the alginate types used. EDS analysis confirmed the presence of $Mn^{2+}$ ions on the surface of the microparticles, paving the way for their application into MEMRI based approaches.

In an embodiment, preparation of in vitro phantoms and imaging at clinical 3T scanner (Magnetom Trio 3T, Siemens). Manganese ($MnCl_2$) was dissolved in water at concentrations ranging from 0.0001 - 80 mM. Serial dilutions of manganese were placed in 1.5 mL Eppendorf tubes with 1 mL volume of the solution in each tube and imaged using T1 weighted sequence. T1 weighted turbo spin echo sequence was used with the following parameters: imaging matrix=384x384; flip angle=150; echo time=9.3; repetition time=650; slice thickness 1 mm.

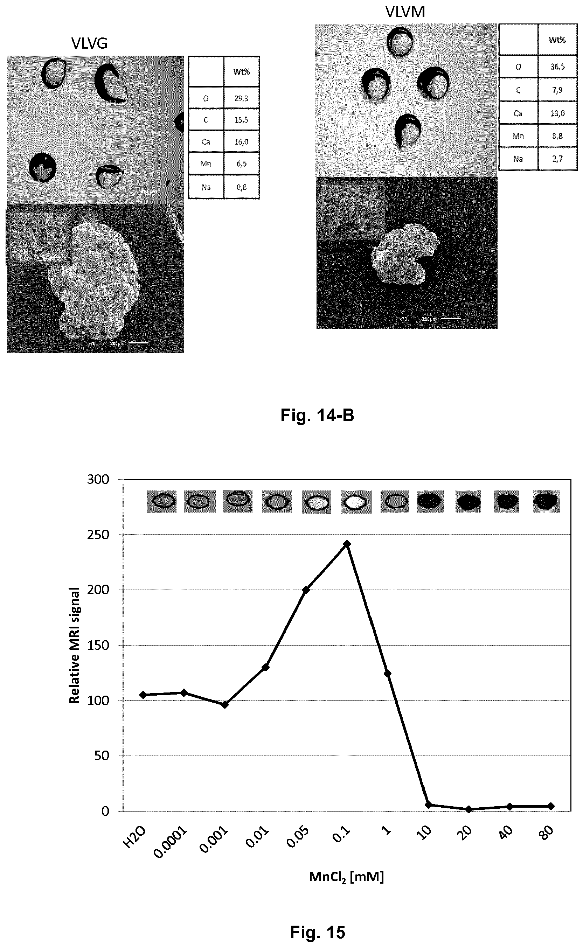

In an embodiment, MRI analysis with signal intensity measurements revealed clear dependence on Mn2+ concentration (Figure 15) at concentrations at 0.001 mM and below signal was at the level of water. At higher concentrations there was clear enhancement of signal that peaked at 0.1 mM concentration. Further increase of the concentration resulted in reduction of signal intensity. Based on these experiments we established Mn2+ concentration of 0.1 mM as optimal for subsequent imaging studies.

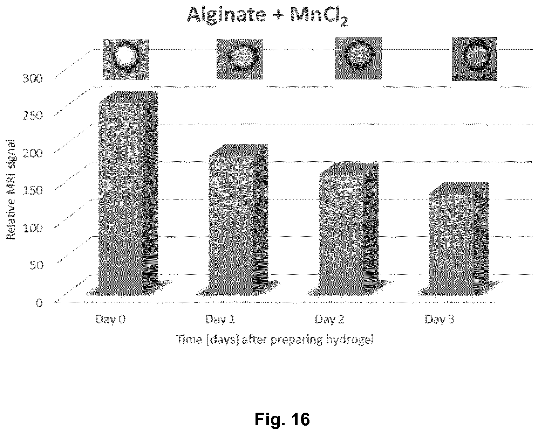

In an embodiment, the Stability of MnCl2 in alginate hydrogels in vitro was analyzed. Low viscosity mannuronic acid based alginate (LVM) and CaM ($>75\mu m$) particles were diluted in 0.9% NaCl to obtain 1% formulations. MnCl2 solution (NaCl) was added to LVM in order to obtain 0.1 mM concentration of Mn2+. Then the LVM/ MnCl2 solution was crosslinked with CaM solution particles to obtain a hydrogel. After 2 minutes of gelation, hydrogel was loaded into the semi permeable insert ($0.47 \text{ cm}^2$ area; $0.4 \mu m$ pore size) and the insert was immersed in artificial cerebrospinal fluid for washing on a rocker for 1, 2 and 3 days. For the relative signal analysis hydrogels were scanned in Magnetom Trio 3T scanner, which revealed gradual reduction of signal intensity over time **(Figure 16)**.

In an embodiment, for the intrathecal transplantation of $Mn^{2+}$ based hydrogels four juvenile Large White domestic pigs with average weight of 45 kg were used. All animal procedures were approved by local ethics committee. Isoflurane anesthetized pigs were positioned on an angiography table and MRI-compatible catheter was introduced through lumbar puncture into intrathecal space. The catheter was advanced to thoracic section of the spinal cord under C-arm x-ray guidance. Subsequently, after securing the catheter in place animal was transferred to MRI suite for interventional injection procedure.

In an embodiment, after acquiring set of baseline MRI scans $Mn^{2+}$ labeled hydrogel was prepared for injection. Low viscosity mannuronic acid-based alginate (LVM) and CaM ($>75 \mu m$) particles were diluted in 0.9% NaCl to obtain 1% formulations. $MnCl_2$ solution (NaCl) was added to LVM to obtain 0.1 mM concentration of $Mn^{2+}$. Then the $LVM/MnCl_2$ solution was crosslinked with CaM solution particles to obtain a hydrogel. After 2 minutes post cross-linking, hydrogel was slowly injected intrathecally via catheter with continues real-time T1 MRI. For real-time imaging of the intervention we used T1 flash sequence with the following parameters: fat saturation; imaging matrix=192x192; flip angle=57; echo time=2.27 ms; repetition time=23 ms; slice thickness 3 mm; temporal resolution=4s.

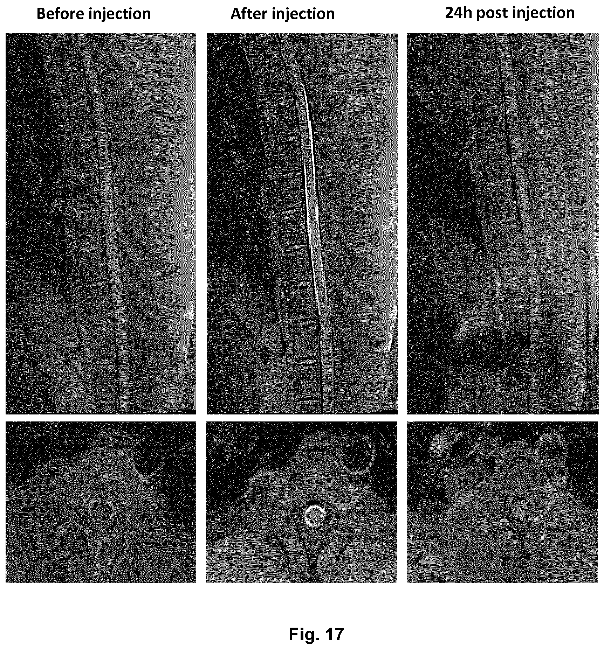

In an embodiment, dynamic imaging during hydrogel infusion revealed hyperintense area corresponding to the injected $Mn^{2+}$ labeled hydrogel. The hyperintensity was first visible at the catheter tip and expanded rostrally and caudally throughout the injection procedure. The area covered by hydrogel formulation ranged between 10-15 cm in tested pigs (Figure 17). Follow-up scans 24h after injection did not reveal any inflammatory responses or evidence for obstructing CSF circulation. Manganese enhancement was at that time not detectable.

The term "comprising" whenever used in this document is intended to indicate the presence of stated features, integers, steps, components, but not to preclude the presence or addition of one or more other features, integers, steps, components or groups thereof.

Where singular forms of elements or features are used in the specification of the claims, the plural form is also included, and vice versa, if not specifically excluded. For example, the term "a polysaccharide" or "the polysaccharide" also includes the plural forms "polysaccharides" or "the polysaccharides," and vice versa. In the claims articles such as "a," "an," and "the" may mean one or more than one unless indicated to the contrary or otherwise evident from the context. Claims or descriptions that include "or" between one or more members of a group are considered satisfied if one, more than one, or all of the group members are present in, employed in, or otherwise relevant to a given product or process unless indicated to the contrary or otherwise evident from the context. The invention includes embodiments in which exactly one member of the group is present in, employed in, or otherwise relevant to a given product or process. The invention also includes embodiments in which more than one, or all of the group members are present in, employed in, or otherwise relevant to a given product or process.

Furthermore, it is to be understood that the invention encompasses all variations, combinations, and permutations in which one or more limitations, elements, clauses, descriptive terms, etc., from one or more of the claims or from relevant portions of the description is introduced into another claim. For example, any claim that is dependent on another claim can be modified to include one or more limitations found in any other claim that is dependent on the same base claim.

Furthermore, where the claims recite a composition, it is to be understood that methods of using the composition for any of the purposes disclosed herein are included, and methods of making the composition according to any of the methods of making disclosed herein or other methods known in the art are included, unless otherwise indicated or unless it would be evident to one of ordinary skill in the art that a contradiction or inconsistency would arise.

Where ranges are given, endpoints are included. Furthermore, it is to be understood that unless otherwise indicated or otherwise evident from the context and/or the understanding of one of ordinary skill in the art, values that are expressed as ranges can assume any specific value within the stated ranges in different embodiments of the invention, to the tenth of the unit of the lower limit of the range, unless the context clearly dictates otherwise. It is also to be understood that unless otherwise indicated or otherwise evident from the context and/or the understanding of one of ordinary skill in the art, values expressed as ranges can assume any subrange within the given range, wherein the endpoints of the subrange are expressed to the same degree of accuracy as the tenth of the unit of the lower limit of the range.

The disclosure should not be seen in any way restricted to the embodiments described and a person with ordinary skill in the art will foresee many possibilities to modifications thereof.

The above described embodiments are combinable.

The following claims further set out particular embodiments of the disclosure:

[1] J. Silva-Correia, J. M. Oliveira, S. G. Caridade, J. T. Oliveira, R. A. Sousa, J. F. Mano, et al., "Gellan gum-based hydrogels for intervertebral disc tissue-engineering applications.," Journal of tissue engineering and regenerative medicine, vol. 5, pp. e97-107, 2011.

[2] S. Vieira, S. Vial, F. R. Maia, M. Carvalho, R. L. Reis, P. L. Granja, et al., "Gellan gum-coated gold nanorods: an intracellular nanosystem for bone tissue engineering," RSC Adv., vol. 5, pp. 77996-78005, 2015.

[3] R. Lozano, L. Stevens, B. C. Thompson, K. J. Gilmore, R. Gorkin, E. M. Stewart, et al., "3D printing of layered brain-like structures using peptide modified gellan gum substrates," Biomaterials, vol. 67, pp. 264-273, 2015.

[4] L. R. Stevens, K. J. Gilmore, G. G. Wallace, and M. In Het Panhuis, "Tissue engineering with gellan gum," Biomater Sci, vol. 4, pp. 1276-90, Aug 16 2016.

[5] Y. A. Morch, I. Sandvig, O. Olsen, I. Donati, M. Thuen, G. Skjak-Braek, et al., "Mn-alginate gels as a novel system for controlled release of Mn2+ in manganese-enhanced MRI," Contrast Media Mol Imaging, vol. 7, pp. 265-75, Mar-Apr 2012.

[6] S. Pina, J. M. Oliveira, and R. L. Reis, "Natural-Based Nanocomposites for Bone Tissue Engineering and Regenerative Medicine: A Review," Adv. Mater., vol. 27, no. 7, pp. 1143-1169, Feb. 2015.

[7] S. Vieira, A. da Silva Morais, J. Silva-Correia, J. M. Oliveira, and R. L. Reis, "Natural-Based Hydrogels: From Processing to Applications," in Encyclopedia of Polymer Science and Technology, no. M, Hoboken, NJ, USA: John Wiley & Sons, Inc., 2017, pp. 1-27.

[8] S. C. Y. Lin, Y. Wang, D. F. Wertheim, and A. G. A. Coombes, "Production and in vitro evaluation of macroporous, cell-encapsulating alginate fibres for nerve repair," Mater. Sci. Eng. C, vol. 73, pp. 653-664, 2017.

[9] E. KOH, Y. C. JUNG, H.-M. WOO, and B.-J. KANG, "Injectable alginate-microencapsulated canine adipose tissue-derived mesenchymal stem cells for enhanced viable cell retention," J. Vet. Med. Sci., vol. 79, no. 3, pp. 492-501, 2017.

[10] H. Daemi and M. Barikani, "Synthesis and characterization of calcium alginate nanoparticles, sodium homopolymannuronate salt and its calcium nanoparticles," Sci. Iran., vol. 19, no. 6, pp. 2023-2028, 2012.

[11] Q. Wang, S. Liu, F. Yang, L. Gan, X. Yang, and Y. Yang, "Magnetic alginate microspheres detected by MRI fabricated using microfluidic technique and release behavior of encapsulated dual drugs," Int. J. Nanomedicine, vol. 12, pp. 4335-4347, 2017.

[12] Ý. A. Mørch, I. Sandvig, Ø. Olsen, I. Donati, M. Thuen, G. Skjåk-Bræk, O. Haraldseth, and C. Brekken, "Mn-alginate gels as a novel system for controlled release of Mn 2+ in manganese-enhanced MRI," Contrast Media Mol. Imaging, vol. 7, no. 2, pp. 265-275, 2012.

**Claims**

1. An ionically crosslinked hydrogel comprising:

   a biocompatible polysaccharide,
   manganese as the ionic crosslinking agent,
   wherein the concentration of manganese varies between 0.001-9 mM.

2. The ionically crosslinked hydrogel according to the previous claims wherein the concentration of manganese varies between 0.01-5 mM.

3. The ionically crosslinked hydrogel according to the previous claims wherein the concentration of manganese varies between 0.05-1 mM, preferably 0.1 mM.

4. The ionically crosslinked hydrogel according to the previous claims further comprising a second ionic crosslinking agent selected calcium, barium or mixtures thereof.

5. The ionically crosslinked hydrogel according to the previous claims wherein the concentration of biocompatible polysaccharide varies between 0.25-10% (wt/V$_{hydrogel}$), preferably between 0.5-2 % (wt/V$_{hydrogel}$), preferably between 0.75-1 % (wt/V$_{hydrogel}$).

6. The ionically crosslinked hydrogel according to the previous claim wherein the biocompatible polysaccharide is selected from a list consisting of: alginate, hyaluronan, gellan gum, or mixtures thereof, preferably a mixture of hyaluronan and gellan gum.

7. The ionically crosslinked hydrogel according to any one of the previous claims wherein the mass ratio of gellan gum: hyaluronan varies between 100:0 to 75:25, in particular substantially 50:50.

8. The ionically crosslinked hydrogel according to any one of the previous claims wherein the gellan gum is a methacrylated gellan gum.

9. The ionically crosslinked hydrogel according to any one of the previous claim wherein the hydrogel the complex viscosity at 37°C varies from 0.08 to 630 Pa.s, preferably from 2 to 100 Pa.s.

10. The ionically crosslinked hydrogel according to any one of the previous claims for use in medicine or veterinary.

11. The ionically crosslinked hydrogel according to the previous claims for use as a drug delivery agent or as a photodynamic therapeutic agent, in particular as a magnetic resonance imaging agent, manganese enhanced-MRI.

12. The ionically crosslinked hydrogel according to the previous claims for use in the treatment, surgery or diagnostic of a diseases by manganese enhanced-MRI.

13. The ionically crosslinked hydrogel according to the previous claims for use in the treatment, surgery or diagnostic of a neoplasia or neurodegenerative disease, in particular cancer or amyotrophic lateral sclerosis.

14. A pharmaceutical composition comprising the ionically crosslinked hydrogel described in any of the previous claims and a pharmaceutical acceptable excipient and/or an active substance or a biomolecule in a therapeutic amount.

15. The composition according to any one of the claims 12-13 wherein the composition is administrated by oral, parenteral, intramuscular, intranasal, sublingual or intratracheal route; in particular an injectable composition.

16. Disc, fibres or microparticles comprising the ionically crosslinked hydrogel or the composition described in any one of the previous claims.

17. A kit comprising the ionically crosslinked hydrogel or the composition described in any one of the previous claims.

**Fig. 1**

**Fig. 2**

Fig. 3

A **Cell viability**

B **DNA quantification**

C

D

**Fig. 4**

GG-MA / HA Ratio

Fig. 5

**GG-MA / HA Ratio**

Fig. 6

GG-MA / HA Ratio

Fig. 7

**Fig. 8**

Fig. 9

**Fig. 10**

**Fig. 11**

**Fig. 12**

**Fig. 13**

LVG

|  | Wt% |
|---|---|
| O | 28,7 |
| C | 20,7 |
| Ca | 13,0 |
| Mn | 7,7 |
| Na | 4,4 |

MVG

|  | Wt% |
|---|---|
| O | 29,1 |
| C | 13,6 |
| Ca | 14,2 |
| Mn | 8,8 |
| Na | 2 |

LVM

|  | Wt% |
|---|---|
| O | 29,8 |
| C | 15,4 |
| Ca | 12,4 |
| Mn | 7,0 |
| Na | 4,4 |

MVM

|  | Wt% |
|---|---|
| O | 39,4 |
| C | 9,7 |
| Ca | 13,5 |
| Mn | 7,8 |
| Na | 1,2 |

**Fig. 14-A**

VLVG

| | Wt% |
|---|---|
| O | 29,3 |
| C | 15,5 |
| Ca | 16,0 |
| Mn | 6,5 |
| Na | 0,8 |

VLVM

| | Wt% |
|---|---|
| O | 36,5 |
| C | 7,9 |
| Ca | 13,0 |
| Mn | 8,8 |
| Na | 2,7 |

Fig. 14-B

Fig. 15

**Fig. 16**

**Before injection**   **After injection**   **24h post injection**

**Fig. 17**

**EP 3 501 553 A1**

Europäisches
Patentamt
European
Patent Office
Office européen
des brevets

# EUROPEAN SEARCH REPORT

Application Number

EP 17 20 9876

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | MØRCH YRR A ET AL: "Mn-alginate gels as a novel system for controlled release of Mn2+ in manganese-enhanced MRI.", CONTRAST MEDIA & MOLECULAR IMAGING, vol. 7, no. 2, March 2012 (2012-03), pages 265-275, XP002781510, ISSN: 1555-4317 * abstract * * page 267 * * page 273 * ----- | 1-17 | INV. A61K49/18 A61K47/69 A61K9/00 A61K9/06 A61K47/36 A61K9/16 A61K9/70 |
| X | AKIHIRO TSUTSUMI ET AL: "ESR studies of Mn(II) binding to gellan and carrageenan gels", FOOD HYDROCOLLOIDS, vol. 7, no. 5, 1 December 1993 (1993-12-01), pages 427-434, XP055478840, ISSN: 0268-005X, DOI: 10.1016/S0268-005X(09)80238-5 * abstract * * page 427 - page 428 * * figures * ----- | 1-17 | |
| X | KAWAHARA S ET AL: "Interactions of paramagnetic metal ions with gellan gum studied by ESR and NMR methods", CARBOHYDRATE POLYMERS, vol. 30, no. 2, 1 January 1996 (1996-01-01), pages 129-133, XP004062696, ISSN: 0144-8617, DOI: 10.1016/S0144-8617(96)00064-1 * abstract * * page 131 * * figures * ----- -/-- | 1-17 | TECHNICAL FIELDS SEARCHED (IPC) A61K |

The present search report has been drawn up for all claims

| Place of search | Date of completion of the search | Examiner |
|---|---|---|
| The Hague | 30 May 2018 | Dullaart, Anwyn |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

28

Europäisches
Patentamt

European
Patent Office

Office européen
des brevets

**EUROPEAN SEARCH REPORT**

Application Number

EP 17 20 9876

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 355 566 B1 (UNIV TEXAS [US]) 28 November 2012 (2012-11-28) * examples * * claims * ----- | 1-17 | |
| X | ADRIEL BORTOLIN ET AL: "Investigação do processo de absorção de água de hidrogéis de polissacarídeo: efeito da carga iônica, presença de sais, concentrações de monômero e polissacarídeo", POLIMEROS: CIENCIA Y TECNOLOGIA, vol. 22, no. 4, 7 August 2012 (2012-08-07), pages 311-317, XP055479319, ISSN: 0104-1428, DOI: 10.1590/S0104-14282012005000046 * abstract * * figures * ----- | 1-17 | |
| A | WO 2013/112491 A1 (UNIV LELAND STANFORD JUNIOR) 1 August 2013 (2013-08-01) * example 2 * ----- | 1-17 | TECHNICAL FIELDS SEARCHED (IPC) |

The present search report has been drawn up for all claims

| Place of search | Date of completion of the search | Examiner |
|---|---|---|
| The Hague | 30 May 2018 | Dullaart, Anwyn |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

## ANNEX TO THE EUROPEAN SEARCH REPORT
## ON EUROPEAN PATENT APPLICATION NO.

EP 17 20 9876

This annex lists the patent family members relating to the patent documents cited in the above-mentioned European search report.
The members are as contained in the European Patent Office EDP file on
The European Patent Office is in no way liable for these particulars which are merely given for the purpose of information.

30-05-2018

| Patent document cited in search report | | Publication date | Patent family member(s) | | Publication date |
|---|---|---|---|---|---|
| EP 1355566 | B1 | 28-11-2012 | AU | 3104102 A | 01-07-2002 |
| | | | CA | 2432797 A1 | 27-06-2002 |
| | | | DK | 1355566 T3 | 04-03-2013 |
| | | | EP | 1355566 A2 | 29-10-2003 |
| | | | HK | 1060283 A1 | 05-07-2013 |
| | | | JP | 2004528278 A | 16-09-2004 |
| | | | US | 2005208138 A1 | 22-09-2005 |
| | | | US | 2005227910 A1 | 13-10-2005 |
| | | | WO | 0249501 A2 | 27-06-2002 |
| WO 2013112491 | A1 | 01-08-2013 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **J. SILVA-CORREIA ; J. M. OLIVEIRA ; S. G. CARIDADE ; J. T. OLIVEIRA ; R. A. SOUSA ; J. F. MANO et al.** Gellan gum-based hydrogels for intervertebral disc tissue-engineering applications. *Journal of tissue engineering and regenerative medicine,* 2011, vol. 5, e97-107
- **S. VIEIRA ; S. VIAL ; F. R. MAIA ; M. CARVALHO ; R. L. REIS ; P. L. GRANJA et al.** Gellan gum-coated gold nanorods: an intracellular nanosystem for bone tissue engineering. *RSC Adv.,* 2015, vol. 5, 77996-78005
- **R. LOZANO ; L. STEVENS ; B. C. THOMPSON ; K. J. GILMORE ; R. GORKIN ; E. M. STEWART et al.** 3D printing of layered brain-like structures using peptide modified gellan gum substrates. *Biomaterials,* 2015, vol. 67, 264-273
- **L. R. STEVENS ; K. J. GILMORE ; G. G. WALLACE ; M. IN HET PANHUIS.** Tissue engineering with gellan gum. *Biomater Sci,* 16 August 2016, vol. 4, 1276-90
- **Y. A. MORCH ; I. SANDVIG ; O. OLSEN ; I. DONATI ; M. THUEN ; G. SKJAK-BRAEK et al.** Mn-alginate gels as a novel system for controlled release of Mn2+ in manganese-enhanced MRI. *Contrast Media Mol Imaging,* March 2012, vol. 7, 265-75
- **S. PINA ; J. M. OLIVEIRA ; R. L. REIS.** Natural-Based Nanocomposites for Bone Tissue Engineering and Regenerative Medicine: A Review. *Adv. Mater.,* February 2015, vol. 27 (7), 1143-1169

- Natural-Based Hydrogels: From Processing to Applications. **S. VIEIRA ; A. DA SILVA MORAIS ; J. SILVA-CORREIA ; J. M. OLIVEIRA ; R. L. REIS.** Encyclopedia of Polymer Science and Technology. John Wiley & Sons, Inc, 2017, 1-27
- **S. C. Y. LIN ; Y. WANG ; D. F. WERTHEIM ; A. G. A. COOMBES.** Production and in vitro evaluation of macroporous, cell-encapsulating alginate fibres for nerve repair. *Mater. Sci. Eng. C,* 2017, vol. 73, 653-664
- **E. KOH ; Y. C. JUNG ; H.-M. WOO ; B.-J. KANG.** Injectable alginate-microencapsulated canine adipose tissue-derived mesenchymal stem cells for enhanced viable cell retention. *J. Vet. Med. Sci.,* 2017, vol. 79 (3), 492-501
- **H. DAEMI ; M. BARIKANI.** Synthesis and characterization of calcium alginate nanoparticles, sodium homopolymannuronate salt and its calcium nanoparticles. *Sci. Iran.,* 2012, vol. 19 (6), 2023-2028
- **Q. WANG ; S. LIU ; F. YANG ; L. GAN ; X. YANG ; Y. YANG.** Magnetic alginate microspheres detected by MRI fabricated using microfluidic technique and release behavior of encapsulated dual drugs. *Int. J. Nanomedicine,* 2017, vol. 12, 4335-4347
- **Ý. A. MØRCH ; I. SANDVIG ; Ø. OLSEN ; I. DONATI ; M. THUEN ; G. SKJÅK-BRÆK ; O. HARALDSETH ; C. BREKKEN.** Mn-alginate gels as a novel system for controlled release of Mn 2+ in manganese-enhanced MRI. *Contrast Media Mol. Imaging,* 2012, vol. 7 (2), 265-275